# EUROPÄISCHE PATENTSCHRIFT

(11) **EP 1 448 219 B1**
(45) Veröffentlichungstag und Bekanntmachung des Hinweises auf die Patenterteilung: **22.03.2006**
(21) Anmeldenummer: 02803801.6
(22) Anmeldetag: 27.11.2002
(51) Int. Cl.: A61K 38/00, A61K 35/20, A61K 31/5377, A61P 27/08

(54) **ANTIGLAUKOMATOSES MITTEL UND DESSEN VERWENDUNG**
ANTIGLAUCOMATOUS AGENT AND USE THEREOF
PRODUIT ANTIGLAUCOMATEUX ET SON UTILISATION

(30) Priorität: 27.11.2001 DE 10158037
(43) Veröffentlichungstag der Anmeldung: 25.08.2004
(73) Patentinhaber: Kosmas KG, 22848 Norderstedt (DE)
(72) Erfinder: GÖRNE, Martin, 22337 Hamburg (DE)
(74) Vertreter: Reitstötter - Kinzebach
(86) Internationale Anmeldenummer: PCT/EP2002/013386
(87) Internationale Veröffentlichungsnummer: WO 2003/045417

(56) Entgegenhaltungen:
- WO-A-86/04217
- DE-A- 3 829 552
- DE-A- 3 922 453

## Beschreibung

Die vorliegende Erfindung betrifft pharmazeutische Mittel auf Basis antiglaukomatöser Wirkstoffe sowie deren Verwendung zur Herstellung eines Medikaments zur Behandlung von Glaukomen.

Lactalbuminhydrolysate werden seit einiger Zeit als Diätnahrungsmittel verwendet. Beispielsweise wird ein Peptidgemisch aus Lactalbumin, Fleisch und Soja bei Maldigestion, Malabsorption, bei konsumierenden Prozessen und zum Aufbau verwendet. Lactalbuminhydrolysate zeichnen sich dadurch aus, dass sie sehr leicht aufgenommen werden können und dem Stoffwechsel in kürzester Zeit zur Verfügung stehen. Insbesondere die niedermolekulare Fraktion ist für die Resorption und weitere Verwertung im Stoffwechsel besonders gut geeignet.

Darüber hinaus wurde auch bereits darüber berichtet, dass ausgehend von Molkenprotein, Lactalbumin, α-Lactalbumin, Lactoferrin, β-Lactoglobulin, Lysozym oder Serumalbumin Abbauprodukte gewonnen werden können, die pharmakologische Wirkungen besitzen sollen. So erwähnen die DE-A 39 22 453 und die PCT/EP86/00016 (WO 86/04217) beispielsweise eine analgetische, antiglaukomatöse oder antimutagene Wirksamkeit. Es wird daher empfohlen, die Abbauprodukte zur Behandlung von schmerzhaften Entzündungen aller Art, Neurodermitis, Arthritis, Rheuma, aber auch von Glaukomen zu verwenden. Auch die gemäß DE 38 29 552 A1 erhältlichen Milchbestandteile sollen charakteristische pharmakologische Eigenschaften besitzen und zur Behandlung von Neurodermitis, Allergien, Glaukoma und zur Immunstimulierung brauchbar sein. Allerdings ist die pharmakologische Wirksamkeit dieser Lactalbuminhydrolysate heftig umstritten. Sie konnte auch in der für die klinische Anwendung geforderten Weise nicht belegt werden.

Glaukome sind Erkrankungen des Auges, deren gemeinsames Kennzeichen darin besteht, zu einem erhöhten Augeninnendruck zu führen. Prinzipiell unterscheidet man zwischen Glaukomen mit verlegtem Kammerwinkel, den Winkelblockglaukomen, und Glaukomen mit offenem Kammerwinkel, den Offenwinkelglaukomen. Ursache der sogenannten Zuflussglaukome ist eine Überproduktion von Kammerwasser, während den sogenannten Abflussglaukomen eine Verschlechterung des Kammerwasserabfluss zugrunde liegt. In der Praxis sind Zuflussglaukome selten. Von wesentlich größerer praktischer Bedeutung sind Abflussglaukome.

Eine mögliche Therapieform stellen chirurgische Eingriffe dar, deren Ziel es ist, einen ausreichenden Abfluss von Kammerwasser zu gewährleisten. Allerdings sind derartige Eingriffe generell mit einem erhöhten Operationsrisiko, insbesondere einem relativ hohen Erblindungsrisiko, auch als Spätkomplikation, verbunden. Fistelbildenden Eingriffen, wie der Goniotrepanation ist darüber hinaus noch das fortgesetzte Risiko einer über die Fistel in das Auge gelangenden Infektion zu eigen. Laserkoagulative Eingriffe, wie die Cyclokryokoagulation, Ciliarkörperexzision sowie die Koagulation im Bereich des Trabekelwerks, haben eine Vernarbung und eine damit einhergehende Verkleinerung des vorhandenen Abflusses zum Nachteil.

Vielfach ist eine dauerhafte Regulierung des Augeninnendrucks mit den gängigen chirurgischen Eingriffen nicht möglich. So erhöht sich mit fortschreitendem Glaukom der Abflusswiderstand des verbleibenden Trabekelwerks und es stellt sich infolgedessen die pathologische Augendruckerhöhung wieder ein.

Aus den vorstehend genannten Gründen werden nicht nur die Offenwinkelglaukome, sondern auch die Winkelblockglaukome zwecks Augeninnendrucksenkung medikamentös behandelt. Ziel der medikamentösen Therapie ist es, den Augendruck so lange wie möglich einzuregulieren, um den Zeitpunkt eines chirurgischen antiglaukomatösen Eingriffs hinauszuschieben bzw. diesen überhaupt zu ermöglichen. Darüber hinaus gibt es Winkelblockglaukome, denen einen operativen Eingriff als nicht erfolgversprechend erscheinen lassende, anatomische Veränderungen zugrunde liegen. In diesen Fällen ist auch beim Winkelblockglaukom eine dauerhafte medikamentöse augendrucksenkende Therapie angezeigt.

Bekannte antiglaukomatöse Wirkstoffe mit augendrucksenkender Wirkung finden sich unter den Muscarinrezeptor-Agonisten, den Betarezeptorenblockern, den Carboanhydrasehemmern und weiteren Wirkstoffen mit anerkannter antiglaukomatöser Wirkung, wie Guanethidin und Latanoprost.

Beispielsweise sind in der FR 7927070 antiglaukomatöse Mittel beschrieben, die Acetazolamid oder Methazolamid in einem wässrigen Milieu mit einem pH-Wert zwischen 6 und 9,5 enthalten. Der pH-Wert kann beispielsweise mit einem Aminosäure-haltigen Puffer eingestellt werden. Eine gefriergetrocknete Zubereitung von Acetylcholin oder einem Salz davon, einer Aminosäure und gewünschtenfalls weiteren Hilfsstoffen ist in JP-A 1228913 beschrieben. Allerdings entfalten diese augendrucksenkenden Mittel vielfach unerwünschte Nebenwirkungen. So können Muscarinrezeptor-Agonisten eine Verengung der Pupille (Miosis) und die damit einhergehenden Störungen des Sehvermögens hervorrufen. Betarezeptorenblocker können Herz-Rhythmus-Störungen bewirken und die Tränensekretion (Sicca-Syndrom) herabsetzen. Auch die oberflächenanalgetische Wirkung der Betarezeptorenblocker ist nachteilig, da kleinere Verletzungen der Hornhaut nicht wahrgenommen werden können und Kontaktlinsen nicht getragen werden sollten. Auch die Carboanhydrasehemmer haben vielfältige unerwünschten Nebenwirkungen.

Da die medikamentöse Therapie in den meisten Fällen über einen langen Zeitraum fortgesetzt und oft sogar lebenslänglich durchgeführt werden muss, sind möglichst geringe Wirkstoffdosen wünschenswert, um die Nebenwirkungen auf ein erträgliches Maß zu ( reduzieren.

Allerdings verlieren die augendrucksenkenden Mittel im Laufe der Behandlung nach und nach ihre drucksenkende Hauptwirkung. Deshalb ist es oft erforderlich, die Dosierung zu erhöhen, wodurch die unerwünschten Nebenwirkungen zunehmen. Beispielsweise kann ein Glaucoma chronicum simplex zunächst behandelt werden, indem man täglich zwei Tropfen einer 0,1 %-igen Timolollösung in den Bindehautsack tropft. Diese anfängliche Timololdosis muss dann im. Verlauf der Behandlung ständig erhöht werden, beispielsweise durch Verabreichung der üblicherweise angebotenen konzentrierteren 0,25 %-igen oder gar 0,5 %-igen Timolollösung. Vielfach wird auch empfohlen, bei nachlassender augendrucksenkender Wirkung eine Kombination verschiedener drucksenkender Substanzen einzusetzen. Allerdings kann auch hier im weiteren Verlauf der Behandlung eine Erhöhung der Einzeldosen erforderlich sein.

Der Erfindung liegt daher die Aufgabe zugrunde, möglichst nebenwirkungsarme antiglaukomatöse Mittel zur Verfügung zu stellen.

Es wurde nun gefunden, daß die Wirkung bekannter antiglaukomatöser Wirkstoffe erheblich verstärkt werden kann, wenn man sie in Kombination mit Lactalbuminhydrolysaten verabreicht.

Gegenstand der vorliegenden Erfindung sind daher pharmazeutische Mittel auf Basis wenigstens eines antiglaukomatösen Wirkstoffs, die dadurch gekennzeichnet sind, daß sie Lactalbuminhydrolysat oder eine Lactalbuminhydrolysat-Peptid enthaltende Fraktion davon enthalten, wobei der antiglaukomatöse Wirkstoff ausgewählt ist unter Acetylcholinchlorid, Carbachol, Bethanicol, Pilocarpin, Aceclidinum, Eserin, Neostigmin, Galanthiamin, Brimonidin, Clonidin, Dipivefrin, Apraclonidin, Carteolol, Timolol, Metipranolol, Betaxolol, Befunolol, Pindolol, Levobunolol, Bupranolol, Brinzolamid, Dorzolamid, Acetazolamid, Methazolamid, Ethoxzolamid, Diclofenamid, Demecarium-Bromid, Diethyl-p-nitrophenylphosphat, Diisopropylfluorophosphat, Ecothiopat-Jodid und Derivaten davon, gegebenenfalls auch in Salzform.

Die erfindungsgemäßen Mittel bieten wesentliche therapeutische Vorteile. Insbesondere können die Antiglaukomatosa wesentlich geringer dosiert werden als es herkömmlicherweise erforderlich ist, um eine bestimmte antiglaukomatöse Wirkung zu erzielen. In der Regel kann die Dosierung des Antiglaukomatosums unter Beibehaltung seiner antiglaukomatösen Hauptwirkung auf etwa 2 Drittel oder gar noch weiter reduziert werden. Dadurch treten weniger, auf das Antiglaukomatosum zurückzuführende Nebenwirkungen auf. Dies ist insbesondere dann von Bedeutung, wenn eine antiglaukoma-töse Behandlung über einen längeren Zeitraum angezeigt ist, wie beispielsweise bei der Behandlung des Offenwinkelglaukoms. Ermöglicht wird dies dadurch, dass die Lactalbuminhydrolysate die antiglaukomatöse Wirkung der Antiglaukomatosa verstärken und damit die Menge, die zur Erzielung einer bestimmten antiglaukomatösen Wirkung benötigt wird, vergleichsweise niedriger ist.

Die Antiglaukomatosa und die Lactalbuminhydrolysate können prinzipiell gemeinsam in einer Formulierung oder getrennt in wenigstens zwei verschiedenen Formulierungen verabreicht werden. Die letztere Möglichkeit beinhaltet sowohl die gleichzeitige als auch die zeitlich beabstandete, d.h. zu unterschiedlichen Zeitpunkten erfolgende, Verabreichung. Bevorzugt ist die gleichzeitige Verabreichung insbesondere in Form einer gemeinsamen Formulierung.

Lactalbumine sind allgemein als Bestandteil der Milch und insbesondere der Molkenproteine bekannt. Es handelt sich hierbei um Proteine, die in an sich bekannter Weise meist im Gemisch mit weiteren Milchbestandteilen, also insbesondere Molkenproteinen, wie Lactalbumin, Lactoferrin, ß-Lactoglobulin, Lysozym oder Serumalbumin, gewonnen werden können.

Als Ausgangsmaterial wird frische Rohmilch eines Haustieres, vorzugsweise Kuhmilch verwendet, die auch einer in den Molkereien heute üblichen Erhitzungsbehandlung unterworfen worden sein kann, vorzugsweise aber unbehandelt ist. Sie kann in üblicher Weise, z.B. durch Zentrifugieren, entrahmt worden sein.

Die Abtrennung der Kaseine kann in an sich bekannter Weise erfolgen, beispielsweise mit Hilfe der sogenannten Lab- oder Säurefällung, bei der die Kaseine ausfallen, während die Molkenproteine in Lösung bleiben. Alternativ können die Kaseine mit Hilfe einer Membranfiltration an einer mikroporösen Membran mit einer Porengröße im Bereich von 0,1 bis 0,6 µm, vorzugsweise 0,2 µm abgetrennt werden. Das Permeat (bzw. Filtrat) enthält sämtliche Salze, Milchzucker, Aminosäuren, Oligopeptide und niedermolekulare Peptide, während im Retentat praktisch alle Kaseinbestandteile der Milch und - sofern nicht vorher entrahmt - auch deren Fettbestandteile enthalten sind.

Aus der von Kasein und Fett weitgehend befreiten Milchfraktion können dann Lactalbumine in an sich bekannter Weise, insbesondere durch Ultrafiltration, gewonnen werden. Beispielsweise können die Lactalbumine als Retentat an einer Membran mit einer Abscheidegrenze von 6 bis 10.000 Da erhalten werden. Brauchbar ist beispielsweise das von der Firma Milei vertriebene Produkt Lactalbumin 75.

Einem besonderen Aspekt zufolge werden erfindungsgemäß bevorzugt Lactalbuminhydrolysate oder Fraktionen davon verwendet, deren gewichtsmittleres Molekulargewicht in einem Bereich von etwa 50 bis 1200, vorzugsweise von etwa 60 bis 800 und insbesondere von etwa 80 bis 500 liegt. Besonders bevorzugt sind Lactalbuminhydrolysate, deren Molekulargewichtsverteilung durch ein oberes Molekulargewicht von etwa 760 und insbesondere von 500 begrenzt ist.

Das erfindungsgemäß zu verwendende Lactalbuminhydrolysat ist dadurch erhältlich, dass man Lactalbumin oder ein Lactalbumin enthaltendes Gemisch (Lactalbumin-Präparation) enzymatisch hydrolisiert. Dazu behandelt man eine wässrige Lactalbumin-Suspension mit wenigstens einer Protease und gewünschtenfalls einer Lipase.

Die Hydrolyse erfolgt in der Regel vollständig, d.h. bis zur Beendigung der enzymatischen Reaktion. Je nach Enzym oder Enzymgemisch kann ein Hydrolysegrad von etwa 20% bis etwa 90%, vorzugsweise von etwa 40% bis etwa 80% und insbesondere etwa 75% erzielt werden.

Der Hydrolysegrad kann in an sich bekannter Weise bestimmt und berechnet werden. So ist es möglich, den Fortschritt der Hydrolyse während der Reaktion zu verfolgen. Als geeignet hat sich insbesondere das Formol-Titrationsverfahren erwiesen, wonach die Anzahl freier Aminogruppen durch Titration mit Natriumhydroxid abgeschätzt werden kann.

Die zwecks Hydrolyse wählbaren Bedingungen, wie die eingesetzten Enzyme und deren Aktivität, die Temperatur, der pH-Wert, die Menge an Lactalbumin und die jeweiligen Konzentrationen des Reaktionsgemisches hängen voneinander ab und können auf fachmännische Art und Weise optimiert werden.

Als Protease(n) verwendet man vorzugsweise Papain, Pankreatin, Chymotrypsin und/oder Trypsin. Gegebenenfalls können auch aus Pilzen und/oder Bakterien gewonnene Proteasen eingesetzt werden, insbesondere zusätzlich zu den vorstehend genannten Proteasen. Die Pilzprotease wird insbesondere ausgewählt unter den Proteasen aus Tritirachium-Arten, insbesondere Tritirachium alba, Proteasen aus Aspergillus-Arten, insbesondere Aspergillus saitoi, Aspergillus sojae, Aspergillus oryzae und/oder aus Rhizopus-Arten, insbesondere Newlase, und die Bakterienprotease wird insbesondere ausgewählt unter Proteasen aus Streptomyces-Arten, insbesondere Streptomyces caespitosus, Streptomyces griseus (Pronase E.), aus Bacillus subtilis-Arten, insbesondere Subtilopeptidase A (Carlsberg Subtilisin), und aus Bacillus polymyxa. Als weitere Proteasen können aus Ananas gewinnbare Proteasen wie das Bromelain verwendet werden.

Sollte die Lactalbumin-Präparation noch merkliche Beimengungen an Stärke und stärkeähnlichen Produkten enthalten, ist es vorteilhaft, eine geeignete Amylase, vorzugsweise eine α-Amylase z.B. aus einer Subtilis-Art mitzuverwenden. Das Aktivitätsoptimum einer derartigen Amylase liegt in der Regel bei einem pH-Wert von 5,7 bis 7,2. Die Reaktionstemperatur kann bis zu 75 °C betragen.

Die Mitverwendung einer Lipase ist in der Regel auch nur dann erforderlich, wenn die Lactalbumin-Präparation merkliche Beimengungen an Fetten enthält.

Vorteilhafterweise werden die Enzyme in einer Menge von etwa 0,01 bis 2 Gew.-%, bezogen auf die Suspension, verwendet.

Als Protease verwendet man vorzugsweise Papain oder ein Gemisch von etwa gleichen Gewichtsteilen Pankreatin und Papain.

Ein weiteres vorteilhaftes Proteasengemisch ist ein Gemisch aus etwa gleichen Anteilen Papain, Pankreatin und einer Bakterien- oder Pilzprotease, z.B. der Bakterienprotease Pronase E aus Streptomyces griseus oder dem Pilzprotease-Produkt "Newlase" aus einer Aspergillus-Art. Ebenfalls von Vorteil ist ein Proteasengemisch aus etwa gleichen Anteilen Papain, Pankreatin und Bromelain.

Insbesondere ist es von Vorteil, bezogen auf etwa 50 kg Lactalbumin als Trockensubstanz, etwa 100 bis 1000 g, vorzugsweise etwa 300 bis 700 g und insbesondere etwa 500 g Papain; etwa 100 bis 1000 g, vorzugsweise etwa 300 bis 700 g und insbesondere etwa 500 g Pankreatin; sowie gegebenenfalls etwa 100 bis 1000 g, vorzugsweise etwa 300 bis 700 g insbesondere etwa 500 g einer Bakterien- oder Pilzprotease, insbesondere Newlase, bzw. einer aus Ananas gewinnbaren Protease, insbesondere Bromelain, zu verwenden.

Zweckmäßigerweise geht man von einer etwa 1 bis 10 gew.%-igen und vorteilhafterweise einer 2 bis 5 gew.%-igen Suspension von Lactalbumin in Wasser aus.

Üblich sind Reaktionszeiten im Bereich von Stunden, insbesondere etwa 1 bis 8 Stunden und vorteilhafterweise etwa 2 bis 4 Stunden; erhöhte Reaktionstemperaturen, etwa 30 bis 70 °C und vorteilhafterweise im Bereich des Temperaturoptimums der eingesetzten Enzyme; pH-Werte im leicht sauren bis leicht alkalischen Bereich, insbesondere etwa bei 6,6 bis 7,8 und vorzugsweise im pH-Optimum der eingesetzten Enzyme. Bei Verwendung von Enzym-Gemischen und insbesondere solchen mit unterschiedlichen Temperatur- bzw. pH-Optima, kann man die jeweiligen Enzyme zu unterschiedlichen Zeiten auf das Lactalbumin einwirken lassen, indem man einen Teil des Enzymgemisches zu einem späteren Zeitpunkt dem Reaktionsgemisch zusetzt und die Bedingungen entsprechend anpasst. Insbesondere kann man die wärmebeständigeren Enzyme zugeben, nachdem man zunächst bei moderater Temperatur unter Einsatz der weniger wärmebeständigen Enzyme das Lactalbumin zumindest teilweise hydrolisiert hat, und anschießend die Hydrolyse unter Verwendung der wärmebeständigeren Enzyme bei höherer Temperatur fortsetzten. So kann man im vorliegenden Fall zunächst bei Temperaturen im Bereich von 35 bis 38 °C und vorzugsweise bei etwa 37 °C Papain und/oder Pankreatin auf das Lactalbumin einwirken lassen und anschließend die Hydrolyse unter Einwirkung einer geeigneten Bakterien- und/oder Pilzprotease bzw. einer aus Ananas gewinnbaren Protease bei Temperaturen im Bereich von etwa 50 bis 75 °C und vorzugsweise bei etwa 60 °C fortsetzen. Zweckmäßigerweise geht man dazu so vor, dass die Protease dem Reaktionsgemisch zugesetzt und dann die Temperatur allmählich erhöht wird. Insbesondere kann es erwünscht sein, die Temperatur während der noch verbleibenden Reaktionszeit graduell auf die Endtemperatur zu erhöhen.

So wird gemäß einer besonderen Ausführungsform der vorliegenden Erfindung ein Lactalbuminhydrolysat verwendet, das dadurch erhältlich ist, dass man ein Papain und Pankreatin enthaltendes Enzymgemisch bei etwa 35 bis 38 °C 1 bis 3 Stunden auf Lactalbumin in wässriger Suspension einwirken lässt; eine Bakterien- oder Pilzprotease bzw. eine aus Ananas gewinnbare Protease zugibt und die Temperatur innerhalb von 1 bis 4 Stunden auf etwa 60 °C erhöht und das Hydrolysat in an sich bekannter Weise gewinnt.

Zur Gewinnung des Hydrolysats geht man zweckmäßigerweise so vor, dass man zunächst die Enzyme inaktiviert. Dazu kann man das Reaktionsgemisch kurz erhitzen. Üblich sind Temperaturen im Bereich von 80 bis 85 °C, wobei eine Inaktivierung schon nach wenigen Minuten erzielt wird. Alternativ können die Enzyme auch durch Sterilisierung bei Ultrahochtemperaturen inaktiviert werden. Wenige Sekunden bei etwa 130 °C reichen im allgemeinen aus.

Als Folge fallen die Enzyme in der Regel aus und der entstandene Niederschlag wird vorzugsweise nach dem Abkühlen des Reaktionsgemisches entfernt. Dazu kann man das Reaktionsgemisch direkt filtrieren oder zunächst einengen, beispielsweise im Vakuum oder durch Sprühtrocknung, um den Rückstand dann in einem geeigneten Lösungsmittel aufzunehmen und die resultierende Suspension zu filtrieren. Als Lösungsmittel eignen sich in diesem Zusammenhang niedere Alkohole wie Methanol, Isopropanol und vorzugsweise Ethanol bzw. deren Gemische mit Wasser und auch organische Lösungsmittel, insbesondere Chloroform.

Aus 5 g Lactalbumin erhält man auf diese Weise beispielsweise etwa 200 mg Lactalbuminhydrolysat in ethanolischer Lösung, wobei dieses Hydrolysat häufig als Gemisch aus den eigentlichen Lactalbuminpeptiden als Hauptbestandteil und weiteren Nebenbestandteilen anfällt. Art und Menge der Nebenbestandteile, insbesondere Fette, hängen vor allem von dem für die Hydrolyse verwendeten Ausgangsmaterial ab.

Gemäß einer besonderen Ausführungsform der vorliegenden Erfindung verwendet man bestimmte Fraktionen aus Lactalbuminhydrolysaten, die insbesondere aus den zuvor beschriebenen durch Extraktion erhältlich sind.

Insbesondere bevorzugt ist es, vor der Extraktion der Lactalbuminhydrolysate diese zunächst zu entfetten. Hierzu eignen sich in bekannter Weise Lösungsmittel wie beispielsweise Petroläther, die zweckmäßigerweise in einer kontinuierlichen, erschöpfenden Extraktion zur Entfernung der Fettbestandteile eingesetzt werden können.

Als Extraktionsmittel eignen sich vorwiegend polare Lösungsmittel, wie niedere Alkohole, insbesondere Methanol, Ethanol, Isopropanol und deren Gemische mit Wasser.

Bevorzugte Fraktionen sind dadurch erhältlich, dass man Lactalbuminhydrolysat mit absolutem Ethanol extrahiert, filtriert und das Filtrat gewinnt. Die ethanolische Lösung enthält eine Lactalbuminhydrolysat-Fraktion A (Ethanol-Extrakt), die durch Einengen als Feststoff gewonnen werden kann. Diese Extraktion des vorzugsweise entfetteten Lactalbuminhydrolysats mit Ethanol erfolgt vorzugsweise durch kontinuierliche, erschöpfende Extraktion. Dies beinhaltet auch die Abtrennung nicht extrahierbarer, d.h. in Ethanol unlöslicher Bestandteile unter Gewinnung einer Extraktlösung, die anschließend zur Trockne eingeengt werden kann, um die auf diese Art extrahierbare Fraktion des Lactalbuminhydrolsyats als Feststoff zu gewinnen.

Besonders bevorzugte Fraktionen sind dadurch erhältlich, daß man Lactalbuminhydrolysat oder Fraktionen davon mit Isopropanol extrahiert, filtriert und das Filtrat gewinnt. Die isopropanolische Lösung enthält eine Lactalbuminhydrolysat-Fraktion B (Isopropanol-Extrakt), die durch Einengen als Feststoff gewonnen werden kann. Ein bevorzugter Isopropanol-Extrakt ist dadurch erhältlich, dass man den mittels Ethanol-Extraktion erhaltenen Rückstand A einsetzt und entsprechend mit Isopropanol extrahiert (Lactalbuminhydrolysat-Fraktion B1).

Dazu kann Lactalbuminhydrolysat und insbesondere die mittels der zuvor beschriebenen Ethanolextraktion gewonnene Fraktion A in Isopropanol aufgenommen werden, so dass ein gut rührbares Gemisch entsteht. Beispielsweise kann das Lactalbuminhydrolysat bzw. eine Fraktion davon mit etwa dem 10-fachen Volumen Isopropanol verrührt werden. In der Regel erhält man eine Suspension, die zur Abtrennung von Feststoffen filtriert wird. Es hat sich als zweckmäßig erwiesen, das Gemisch zunächst einige Stunden bis wenige Tage vorzugsweise in der Kälte aufzubewahren und dann die resultierende Feststoffphase abzufiltrieren. Etwa 2 Tage bei einer Temperatur von 2 °C bis 4 °C führen zu guten Ergebnissen. Anschließend wird das Filtrat zur Trockne eingeengt, wobei obigen Ausführungen zur Einengung des Ethanolextraktes entsprechend verfahren werden kann. Darüber hinaus hat es sich als zweckmäßig erwiesen, zur Entfernung von Isopropanolresten den Rückstand wiederholt in Ethanol aufzunehmen und durch Einengen von Lösungsmitteln zu befreien. Beispielsweise werden durch dreimaliges Aufnehmen mit ausreichend Ethanol und anschließendem Einengen gute Ergebnisse erzielt.

Besonders bevorzugte Fraktionen sind dadurch erhältlich, daß man den mittels Ethanol-Extraktion erhaltenen Rückstand A in absolutem Ethanol aufnimmt und mit Wasser einen Ethanolgehalt von etwa 20 bis 60 Vol.-%, vorzugsweise von etwa 30 bis 50 Vol.-% und insbesondere von etwa 40 Vol.-%, einstellt. In der Regel erhält man eine Suspension, deren Feststoffphase durch Filtration abgetrennt werden kann. Dazu ist es zweckmäßig, das gegebenenfalls zunächst noch als Lösung vorliegende Gemisch mehrere Stunden bis wenige Tage, vorzugsweise in der Kälte aufzubewahren und dann abzufiltrieren. Auch hier führen 2 Tage bei 2 °C bis 4 °C zu guten Ergebnissen. Die wäßrig-ethanolische Lösung enthält eine Lactalbuminhydrolysat-Fraktion C, die durch Einengen als Feststoff gewonnen werden kann. Bevorzugte Lactalbuminhydrolysat-Fraktionen sind erhältlich, indem man den mittels Isopropanol-Extraktion erhaltenen Rückstand B und insbesondere B1 einsetzt und entsprechend verfährt, wobei man eine Lactalbuminhydrolysat-Fraktion C1 bzw. insbesondere eine Lactalbuminhydrolysat-Fraktion C2 gewinnt.

In der Regel erfolgt das Einengen zur Trockne in an sich bekannter Weise, z.B. im Vakuum oder durch Sprühtrocknung. Das Einengen kann zweckmäßigerweise durch Verdampfen des Ethanols bei leicht erhöhter Temperatur, beispielsweise bei etwa 40 °C, erfolgen. Gewünschtenfalls wird der gewonnene Rückstand weiter getrocknet, beispielsweise im Vakuum. Andere, ähnlich schonende Verfahren sind ebenfalls geeignet. Für die Entfernung des Isopropanols bzw. wäßrig-alkoholischer Gemische gilt ähnliches.

Die Lactalbuminhydrolysat-Fraktionen sind gekennzeichnet durch einen erhöhten Gehalt an Lactalbuminhydrolysat-Peptiden, einer Fraktion des anfänglich eingesetzten Lactalbuminhydrolysats. Einem besonderen Aspekt zufolge beinhaltet diese Peptidfraktion 2 Petide (Peptid A und Peptid B), deren jeweiliger Anteil an der Peptidfraktion größer ist als der Anteil eines beliebigen weiteren Peptids in dieser Fraktion. Insbesondere handelt es sich bei den beiden Peptiden A und B um Tri- und/oder Tetrapetide. Einem weiteren Aspekt zufolge sind die Peptide A und B durch ein chromatographisches Verhalten gekennzeichnet, wonach sie mittels Umkehrphasen-Chromatographie, z.B. bei Verwendung einer RP 18-Säule (5 µ-Material) durch Elution mit einem Acetonitril/Wasser/Trifluoressigsäure-Gradienten, bei ausreichend unterschiedlichen Verweilzeiten, aufgereinigt werden können.

Dementsprechend können die Peptide A und B aus Lactalbuminhydrolysaten oder Fraktionen davon angereichert und gewonnen werden.

Besondere Lactalbuminhydrolysat-Peptide erfindungsgemäßer Lactalbuminhydrolysate sind einem weiteren Aspekt zufolge gekennzeichnet durch ein Molekülion-Peak (m + H⁺) bei massenspektrometischer TOF-Analyse von m/z = 247, 269, 341 und/oder 399. Von besonderer Bedeutung sind die Peptide mit den Molekülion-Peaks 341 und 399. Besondere Lactalbuminhydrolysat-Peptide sind daher vor allem auszuwählen unter Tripeptiden mit zwei Prolin-Resten sowie Glutamin oder Lysin als weiteren Aminosäurerest und Tetrapeptiden mit einem Leucin- oder einem Isoleucinrest, und einem Prolin-, einem Alanin- und einem Valinrest.

Einem Aspekt zufolge sind überwiegende Anteile erfindungsgemäß zu verwendender Lactalbuminhydrolysate oder Fraktionen davon sowohl in Chloroform als auch in Wasser leicht löslich. Diese Anteile betragen vorzugsweise wenigstens 90 Gew.-%, vorzugsweise wenigstens 95 Gew.-% und insbesondere wenigstens 98 Gew.-% des jeweiligen Lactalbuminhydrolysats bzw. der Fraktion davon.

Zu Antiglaukomatosa im weitesten Sinne gehören Wirkstoffe, die augeninnendrucksenkend wirken. Hierzu gehören bestimmte Muscarinrezeptor-Agonisten, Betarezeptorenblocker, Carboanhydrasehemmer und weitere Wirkstoffe mit bekannter antiglaukomatöser Wirkung.

Besondere erfindungsgemäß verwendbare Antiglaukomatöse Wirkstoffe sind ausgewählt unter:
a) Muscarinrezeptor-Agonisten, wie Acetylcholinchlorid, Carbachol und Bethanicol sowie den entsprechenden Trimethylammoniumderivaten mit weiteren pharmazeutisch verträglichen Gegenionen, Pilocarpin, Aceclidinum, Eserin, Neostigmin, Galanthiamin, Brimonidin, Clonidin, Dipivefrin und Apraclonidin sowie Derivaten davon, gegebenenfalls in Salzform, z.B. Pilocarpin-Nitrat, Pilocarpin-Hydrohydrochlorid, Neostigmin-Bromid, Brimonidin-Tartrat, Clonidin-Hydrochlorid und Dipivefrin-Hydrochlorid;
b) Betarezeptorenblockern, wie Carteolol, Timolol, Metipranolol, Betaxolol, Befunolol, Pindolol, Levobunolol und Bupranolol sowie Derivaten davon, gegebenenfalls in Salzform, z.B. Carteolol-Hydrochlorid, Timolol-Maleat, Timolol-Hydrogenmaleat, Metipranolol-Hydrochlorid, Betaxolol-Hydrochlorid, Befunolol-Hydrochlorid, und Levobunolol-Hydrochlorid;
c) Carboanhydrasehemmern, wie Brinzolamid, Dorzolamid, Acetazolamid, Methazolamid, Ethoxzolamid und Diclofenamid sowie Derivaten davon, gegebenenfalls in Salzform;
d) weiteren Substanzen mit anerkannter antiglaukomatöser Wirkung, wie Demecarium-Bromid, Diethyl-p-nitrophenylphosphat, Diisopropylfluorophosphat und Ecothiopat-Jodid sowie Derivaten davon, gegebenenfalls in Salzform; Guanethidin sowie Derivaten davon, gegebenenfalls in Salzform.

Erfindungsgemäß bevorzugt sind folgende Antiglaukomatosa:

Timolol und Derivate davon, gegebenenfalls auch in Salzform.

Erfindungsgemäß wird dem zu behandelnden Individuum, vorzugsweise einem Säuger, insbesondere einem Menschen und auch einem Nutz- oder Haustier, eine wirksame Menge Lactalbuminhydrolysat oder einer Fraktion davon und eine wirksame Menge wenigstens eines Antiglaukomatosums, in der Regel der pharmazeutischen oder tierarzneilichen Praxis entsprechend formuliert, verabreicht.

In der Regel wird täglich, einmalig oder mehrmalig eine geeignete Dosis gegebenenfalls zusammen oder im Wechsel mit anderen Wirkstoffen oder wirkstoffhaltigen Präparaten verabreicht, so dass einem zu behandelnden Individuum eine Tagesdosis von etwa 2 mg bis 100 g, vorzugsweise von etwa 10 mg bis 80 g, vorteilhafterweise von etwa 20 mg bis 60 g, und insbesondere von etwa 50 mg bis 60 g Lactalbuminhydrolysat bei oraler Gabe, von etwa 0,5 mg bis 80 g, vorzugsweise von etwa 8 mg bis 70 g, vorteilhafterweise von etwa 20 mg bis 70 g, und insbesondere von etwa 30 mg bis 40 g Lactalbuminhydrolysat bei parenteraler Gabe und von etwa 0,5 mg bis 100 g, vorzugsweise von etwa 1 mg bis 80 g, vorteilhafterweise von etwa 5 mg bis 60 g, und insbesondere von etwa 10 mg bis 60 g Lactalbuminhydrolysat bei topischer Anwnendung verabreicht wird. Die zu verabreichenden Mengen an Lactalbuminfraktionen liegen in der Regel unter den vorstehend genannten Dosierungen. So kann die Dosierung für die Lactalbuminhydrolysatbestandteile etwa um den Faktor 5 bis 20 und die Dosierung für die Lactalbuminhydrolysatpeptide etwa um den Faktor 10 bis 30 niedriger gewählt werden.

Dabei kann die Dosierung des Antiglaukomatosums erfindungsgemäß niedriger gewählt werden als es herkömmlicherweise zum Erzielen der antiglaukomatösen Hauptwirkung (Drucksenkung) empfohlen wird. Demnach kann insbesondere die zur Behandlung von Glaukomen empfohlene Tagesdosis zumindest um den Faktor 1,5 und vorteilhafterweise zumindest um den Faktor 2 gesenkt werden. Müssen beispielsweise einem Patienten mit chronischem Offenwinkelglaukom bereits täglich 2 Tropfen einer 0,5 %-igen Timolol-Lösung verabreicht werden, um den Augeninnendruck zu unter 21 mmHg zu senken, so kann erfindungsgemäß die gleiche therapeutische Wirkung mit einer Kombination aus 0,2 mg Lactalbuminhydrolysat-Fraktion C1 oder C2 und 2 Tropfen einer nur 0,1 %-igen Timolol-Lösung erzielt werden.

Das Ausmaß, in dem die Dosis eines Wirkstoffs mit anerkannter antiglaukomatöser Wirkung herabgesetzt werden kann, wenn dieser erfindungsgemäß in Kombination mit Lactalbuminhydrolysat oder einer Fraktion davon verwendet wird, kann insbesondere mit kontrollierten, randomisierten Doppelblindstudien belegt werden. Dazu können beispielsweise klinische Messgrößen herangezogen werden, welche den Verlauf der Therapie des untersuchten Krankheitsbildes nach wissenschaftlichen Erkenntnissen dokumentieren. Ferner können auch Tiermodelle verwendet werden.

Beispielsweise wird die Therapie bei erfindungsgemäßer Verwendung einer Kombination aus Timolol und einer Lactalbuminhydrolysat-Fraktion einerseits und einer 5-fach höheren Thimolol-Dosis ohne Verwendung einer erfindungsgemäßen Lactalbuminhydrolysat-Fraktion für die Behandlung des Glaucoma chronicum simplex mit der Dokumentation des Augeninnendrucks, des Gesichtsfeldes und der fotographischen Kontrolle des Sehnervenkopfes bewertet. Gesichtsfeld- und Sehnervenkopfkontrolle dienen dem Ausschluss eventueller okkulter Augeninnndruckerhöhungen, welche außerhalb des Augendruckmesszeitpunktes auftreten können. Der Grad der Dosisherabsetzung ergibt sich unter Bezugnahme auf Referenzwerte für die Therapie des Glaucoma chronicum simplex.

Wirkstoffmengen und -anteile beziehen sich auf den aktiven Wirkstoff, so dass für Salze und Derivate eine entsprechende Umrechnung zu erfolgen hat.

Die erfindungsgemäßen Mittel eignen sich vor allem zur Behandlung von Glaucoma (auch als Grüner Star bezeichnet). Damit betrifft die vorliegende Erfindung auch die Verwendung von Lactalbuminhydrolysaten oder Fraktionen davon in Kombination mit wenigstens einem Antiglaukomatosum zur Behandlung von Gaukoma.

Die erfindungsgemäßen Mittel zür Glaukombehandlung zielt vor allem auf eine Senkung des Augeninnendrucks ab. Es handelt sich daher um eine symptomatische Behandlungsform. Beim gesunden Auge liegt der durch ein Gleichgewicht aus ständiger Bildung und Abfluss von Kammerwasser gebildete Augeninnendruck im Bereich von etwa 9 mmHg bis etwa 21 mmHg, jeweils applanatorisch gemessen.

Ein erfindungsgemäß zu behandelndes Glaukom ist insbesondere gekennzeichnet durch einen applanatorisch gemessenen Augeninnendruck von mehr als 21 mmHg. Dies gilt vor allem dann, wenn derartig erhöhte Augeninnendruckwerte wiederholt gemessen werden, der. Augeninnendruck also chronisch erhöht ist. Allerdings erfasst die erfindungsgemäße Behandlung auch Niedrigdruckglaukome, bei denen es bereits bei Augeninnendrücken im oberen Normalbereich, z.B. bei applanatorisch gemessenen 20 mmHg, zu glaukomatös bedingte Funktionsausfällen des Auges kommt. Hinweise für beginnende glaukomatös bedingte Funktionsausfälle des Auges, wie Schädigungen des Sehnervenkopfes, insbesondere einer Sehnervenatrophie und/oder einer Papillenexkavation, Gesichtsfeldausfälle, Minderung der Sehkraft und ein grünlicher Reflex der Linse, unterstützen die Glaukom-Diagnose und dienen zur Abgrenzung des Glaukoms von einer okulären Hypertonie, welche bei mäßig erhöhten, insbesondere applanatorisch gemessenen Augendruckwerden von bis zu etwa 26 mmHg, im wesentlichen nicht zu pathologischen Veränderungen, also insbesondere nicht zu den vorstehend geschilderten glaukomatös bedingten Funktionsausfällen des Auges, führt.

Zu den erfindungsgemäß behandelbaren Glaukomen gehören vor allem Glaukome mit offenem Kammerwinkel (Offenwinkelglaukome, auch als Weitwinkelglaukome bezeichnet). Hierzu gehören vor allem das Glaucoma chronicum simplex. Dieses ist insbesondere gekennzeichnet durch einen erhöhten Gehalt an Hyaluronsäure im Trabekelwerk. Ferner finden sich auch Globuline und Plasmazellen im Trabekelwerk. Mit zunehmendem Alter kann es zu einer Verdickung der Trabekelbalken und/oder zu einer Verlegung der intertrabekulären Lamina kommen.

Weitere, erfindungsgemäß behandelbare Offenwinkelglaukome sind das Pigmentglaukom (hier verliert das Pigmentblatt der Iris zunehmend Pigment, welches sich wie Teeblätter in einem Abfluss im Trabekelwerk anreichert und dieses zunehmend verstopft); das Pseudoexfoliationsglaukom, auch als Kapselhäutchen-Glaukom bezeichnet (hier kommt es zur Bildung von pseudoexfoliativem Material, welches sich ähnlich wie beim Pigmentglaukom im Trabekelwerk anreichert und den Abflusswiderstand erhöht); das spätjuvenile Glaukom; Formen des kindlichen Glaukoms (Buphthalmus); insbesondere das Glaucoma juvenile Sautter; das Glaukom bei hoher Myopie; das Glaukom bei Diabetes mellitus; und das Cortison-induzierbare Glaukom.

Zu den erfindungsgemäß behandelbaren Offenwinkelglaukomen zählen ferner auch sekundäre Glaukome, die infolge einer weiteren Erkrankung entstanden sind, beispielsweise Glaukome infolge entzündlicher Veränderungen im Auge; Glaukome infolge von Gefäßneubildungen (neovaskuläres Glaukom); Glaukome infolge einer Uveitis, einer Iridocyclitis, einer Heterochromie-Iridocyclitis Fuchs, eines Kraupa-Posner-Schlossmann-Syndroms und einer Kerato-Uveitis, beispielsweise bei Herpes-simplex-Infektionen oder bei Zoster ophthalmicus; ferner Glaukome infolge eines Kirsch-Effektes durch alpha-Chymotrypsin; Glaukome infolge von Blut in der Vorderkammer; postkontusionelle Glaukome infolge einer Kontusionsdeformität des Kammerwinkels; Makrophagen-Glaukome; Glaukome bei intraokularen Tumoren; Glaukome bei Epitheleinwachsung in den Kammerwinkel; Glaukome bei orbitalen Prozessen infolge eines erhöhten episkleralen Venendrucks, beispielsweise bei einer Myositis oder bei einer arteriovenösen Fistel.

Neben den Offenwinkelglaukomen können auch Winkelblockglaukome erfindungsgemäß behandelt werden. Dadurch kann der Zeitpunkt eines chirurgischen antiglaukomatösen Eingriffs hinausgeschoben oder dieser überhaupt ermöglicht werden (präoperative Senkung des Augeninnendrucks). Insbesondere kann bei bestimmten Formen des Winkelblockglaukoms die erfindungsgemäße medikamentöse Behandlung angezeigt sein, wenn ein chirurgischer Eingriff nicht erfolgversprechend zu sein scheint.

Ein besonderer Aspekt einer Behandlung im erfindungsgemäßen Sinne betrifft die Behandlung chronischer Störungen, also insbesondere des chronischen Offenwinkelglaukoms (Weitwinkelglaukoms).

Die Erfindung betrifft auch die Herstellung von Mitteln zur Behandlung eines Individuums, vorzugsweise eines Säugers, insbesondere eines Menschen und auch eines Nutz- oder Haustieres.

Erfindungsgemäße Mittel basieren in der Regel auf einer Wirkstoffkombination und gegebenenfalls einer Formulierungsgrundlage.

Die Formulierungsgrundlage erfindungsgemäßer Formulierungen enthält physiologisch akzeptable Hilfsstoffe. Physiologisch akzeptabel sind die im Bereich der Pharmazie, der Lebensmitteltechnologie und angrenzenden Gebieten bekanntermaßen verwendbaren Hilfstoffe, insbesondere die in einschlägigen Arzneibüchern (z.B. DAB, Ph. Eur., BP, NF) gelisteten, und auch andere Hilfstoffe, deren Eigenschaften einer physiologischen Anwendung nicht entgegenstehen.

Geeignete Hilfsstoffe können sein: Netzmittel; emulgierende und suspendierende Mittel; konservierende Mittel; Antioxidantien; Antireizstoffe; Chelatbildner; Dragierhilfsmittel; Emulsionsstabilisatoren; Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Cantor-Verlag, 1996, dargestellt ist.

Beispiele geeigneter pharmazeutischer Formulierungen sind feste Arzneiformen, wie Pulver, Puder, Granulate, Tabletten, insbesondere Filmtabletten, Pastillen, Sachets, Cachets, Dragees, Kapseln wie Hart- und Weichgelatinekapseln, Suppositorien oder vaginale Arzneiformen, halbfeste Arzneiformen, wie Salben, Cremes, Hydrogele, Pasten oder Pflaster, sowie flüssige Arzneiformen, wie Lösungen, Emulsionen, insbesondere Öl-in-Wasser-Emulsionen, Suspensionen, beispielsweise Lotionen, Injektions- und Infusionszubereitungen, Augentropfen. Auch implantierte Abgabevorrichtungen können zur Verabreichung erfindungsgemäßer Wirkstoffe verwendet werden. Ferner können auch Liposomen oder Mikrosphären zur Anwendung kommen. Bevorzugt sind flüssige und halbfeste Arzneiformen für die topische und insbesondere intraokuläre Verabreichung, vor allem Lösungen als Tropfen (Augentropfen) und Gele oder Salben zum Eintropfen bzw. Einstreichen in den Bindehautsack, ferner auch feste Arzneiformen.

Die Formulierungen können beispielsweise auf oralem, rektalem, topischem, insbesondere transdermalem, parenteralem, insbesondere subkutanem, intravenösem, intramuskulärem, intraokuläre oder intranasalem Weg verabreicht werden. Bevorzugt ist die topische, insbesondere intraokuläre, sowie die orale und erforderlichenfalls auch intravenöse Verabreichung.

Bei der Herstellung der Formulierungen werden die Wirkstoffe gevöhnlich mit einem geeigneten Hilfsstoff, in diesem Fall auch als Exzipient zu bezeichnen, vermischt oder verdünnt. Exzipienten können feste, halbfeste oder flüssige Materialien sein, die als Vehikel, Träger oder Medium für den Wirkstoff dienen. Die Zunischung weiterer Hilfsstoffe erfolgt erforderlichenfalls in an sich bekannter Weise. Es können Formgebungsschritte, gegebenenfalls in Verbindung mit Mischvörgangen, durchgeführt werden, z.B. eine Granulierung, Komprimierung und ähnliches.

Insbesondere können die Wirkstoffkomponenten gemeinsam formuliert werden. Sie können aber auch zunächst getrennt verarbeitet und anschließend in einer kompartimentierten, z.B. mehrschichtigen Arzneiform zusammengeführt werden. Dadurch kann möglichen Wirkstoffinkompatibilitäten und unterschiedlichen Wirkstoffeigenschaften, wie Bioverfügbarkeit, Stabilität, Löslichkeit und ähnlichem, Rechnung getragen werden.

Neben Kombinationspräparaten betrifft die Erfindung auch entsprechende Monopräparate in Form von Handelspackungen, denen die erfindungsgemäße kombinierte Anwendung zu entnehmen ist.

Die vorliegende Erfindung wird anhand der nachfolgenden Beispiele näher erläutert, ohne darauf beschränkt zu sein.

### Herstellung von Lactalbuminhydrolysat und Fraktionen davon

### Beispiel 1

### Herstellung eines Lactalbuminhydrolysats

5 g Lactalbumin werden in 130 ml Wasser suspendiert, man gibt 50 mg Papain und 50 mg Pankreatin hinzu, erwärmt auf 35-37 °C, rührt bei dieser Temperatur etwa 2 Stunden, gibt 50 mg einer handelsüblichen Pilzprotease, z.B. Newlase, eine Protease aus Rhizopus mit einer Aktivität von ungefähr 0,5 E/mg Masse, hinzu und steigert die Temperatur langsam (innerhalb von 2-3 Stunden) auf 60 °C. Dann erhöht man die Temperatur kurz auf 80 °C, lässt abkühlen, engt die trübe Lösung im Vakuum ein, nimmt den Rückstand in Ethanol auf, filtriert und engt im Vakuum ein. Man erhält etwa 0,2 g Lactalbuminhydrolysat.

### Beispiel 2

### Entfettung des Lactalbuminhydrolysats

100 g des nach Beispiel 1 erhaltenen Lactalbuminhydrolysats werden in einer kontinuierlichen, erschöpfenden Reaktion mit 2 1 Petroläther entfettet bei einer Temperatur von etwa 22 °C.

### Beispiel 3

### Gewinnung einer Lactalbuminhydrolysat-Fraktion A

5 g des nach Beispiel 2 erhaltenen, entfetteten Lactalbuminhydrolysats werden mit 100 ml absolutem Ethanol bei einer Temperatur von 22 °C in einer kontinuierlichen, erschöpfenden Reaktion extrahiert. Der Ethanolextrakt wird bei 40 °C eingedampft und im Vakuum getrocknet.

### Beispiel 4

### Gewinnung einer Lactalbuminhydrolysat-Fraktion B

500 g der nach Beispiel 3 erhaltenen Lactalbuminhydrolysat-Fraktion A werden mit dem 10-fachen Volumen Isopropanol verrührt und 2 Tage bei einer Temperatur von 2 °C bis 4 °C aufbewahrt. Man filtriert oder zentrifugiert und engt die klare Lösung bei 50 °C im Vakuum zur Trockne ein.

Zur Entfernung von Isopropanolresten wird der Rückstand in 2 1 Ethanol aufgenommen und das Ethanol bei 40 °C im Vakuum verdampft. Dieser Vorgang wird 2-3-mal wiederholt. Man erhält ca. 400 g einer Lactalbuminhydrolysat-Fraktion B1 als gelbliches Pulver.

### Beispiel 5

### Gewinnung einer Lactalbuminhydrolysat-Fraktion C

Die von Isopropanolresten nach Beispiel 4 befreite Lactalbuminhydrolysat-Fraktion B1 wird in 2 1 absolutem Ethanol gelöst und durch Zugabe von Wasser auf einen Ethanolgehalt von 40 Vol.-% eingestellt. Anschließend wird die Lösung 2 Tage bei 2-4 °C aufbewahrt. Man filtriert oder zentrifugiert und engt die erhaltene klare Lösung im Vakuum bei 40 °C zur Trockne ein. Man erhält ca. 300 g einer Lactalbuminhydrolysat-Fraktion C2 als gelbliches Pulver.

Die massenspektrometische Analyse der Lactalbuminhydrolysat-Fraktion C2 ergibt folgende Peaks (m/z +/- 0,5; TOF MS ES+):
72,09; 86,10; 98,99; 120,09; 127,08; 149,03; 185,18; 188,08; 199,19; 229,17; 244,18; 247,12; 269,17; 286,99; 288,98; 341,23; 349,01; 360,21; 399,27; 431,25; 453,23; 469,23; 496,35; 525,36; 527,37; 557,35; 565,28; 569,30; 587,26; 587,28; 609,38; 649,35; 654,41; 682,42; 683,36; 723,37; 724,36; 763,40; 764,43; 772,42; 773,44; 797,42; 837,47; 838,49; 860,45; 871,52; 872,51; 889,54; 926,55; 927,51; 948,52; 949,46; 951,53.

Die HPLC-analytische Untersuchung (Umkehrphase RP-18; 5 µ-Material; Elution mit einem Acetonitril/Wasser/Trifluoressigsäure-Gradienten; Detektion bei 205 nm) der Lactalbuminhydrolysat-Fraktion C2 zeigt neben freien Aminosäuren ein Peptidgemisch, das im Oligopeptid-Bereich (Dipeptide bis Decapeptide) zwei Hauptfraktionen umfasst, die, bezogen auf die bei etwa 7,5 min eluierenden hydrophoben Aminosäuren, insbesondere Tryptophan, bei etwa 16 min bzw. etwa 19,5 min eluieren. Diese Hauptfraktionen stehen ihrem chromatographischen Verhalten nach für Tri- oder Tetrapeptide, so dass anzunehmen ist, dass ein wesentlicher Bestandteil der erfindungsgemäßen Lactalbuminhydrolysate aus zwei Peptiden gebildet wird, wobei es sich um Tri- und/oder Tetrapeptide handeln dürfte.

Diese Peptide lassen sich mit einer entsprechenden präparativen HPLC isolieren.

### Pharmazeutische Mittel

### Beispiel 6

| a) Augentropfen mit Timolol: | |
|---|---|
| Timololhydrogenmaleat | 1,37mg |
| Lactalbuminhydrolysat-Fraktion nach Bsp. 5 | 5 mg |
| Benzalkoniumchlorid | 0,05mg |
| Natriumdihydrogenphosphat | |
| Natriummonohydrogenphosphat | |
| Wasser | ad 1 g |

Zur Behandlung von Glaukoma Chronicum simplex sind 1 bis 2 Tropfen täglich in den Bindehautsack zu nehmen.

### Beispiel 7

Es wurden Patienten behandelt, bei denen ein Glaukom vorlag, welches zu glaukomatösen Schäden geführt hat. Alle Patienten waren zuvor mit Timolol 0,5 % Augentropfen, teilweise mit einer Komtinationstherapie von Timolol 0,5 % und einem weiteren antiglaukomatösen Mittel behandelt worden. Die Ausgangslage des Augendrucks wurde unter der bisherigen Therapie ermittelt, indem der Mittelwert der letzten sechs Messungen unter der bisherigen Therapie bestimmt wurde.

Es wurden dann zwei Patientengruppen erfindungsgemäß behandelt. In Gruppe 1 waren Patienten zusammengefasst, die unter maximaler Tropftherapie, d.h. einer Kombinationstherapie mit Timolol 0,5 % und einem weiteren Antiglaukomatosum (z.B. Pilocarpin 2 %, Clonidin 1/4 %, Carbachol 2 %) keine normalen Augendruckwerte aufwiesen. Diese bildeten die Gruppe der dekompensierten Glaukome.

In Gruppe 2 waren Patienten, die bei Behandlung mit Timolol 0,5 % normalisierte Augendruckwerte aufwiesen. Diese bildeten die Gruppe der kompensierten Glaukome.

Die Patienten aus Gruppe 1 und 2 wurden dann mit Augentropfen, die 0,1 % Timolol und 0,5 % Lactalbuminhydrolysat-Fraktion gemäß Beispiel 5 enthielten, zweimal täglich behandelt, indem jeweils ein Tropfen der Lösung in den Bindehautsack eingeträufelt wurde.

Eine dritte, aus 11 Patienten gebildete Gruppe wurde mit Augentropfen, die lediglich 0,5% Lactalbuminhydrolysat-Fraktion gemäß Beispiel 5 enthielten, in der gleichen Art und Weise behandelt.

Der Augeninnendruck wurde anfangs nach einem Tag, nach zwei Tagen, dann wöchentlich kontrolliert. Die Augendruckmessungen erfolgten zu unterschiedlichen Tageszeiten (morgens, mittags, spätnachmittags) mittels Applanationstonometrie.

Insgesamt wurden 18 Patienten mit einem Durchschnittsalter von 56,6 Jahren, von denen 8 männlich und 10 Patienten weiblich waren, 9 Wochen behandelt.

Bei den Patetienten wurde vor der erfindungsgemäßen Behandlung folgende Diagnose gestellt:

| Gl, chr. simplex | postkontusionelles G1 | neovask. G1 | Sekundärgl. bei i.o. Entz. |
|---|---|---|---|
| 9 2 5 | | | 2 |

| Gruppe 1: dekompensiert>21 mm Hg appl | | | |
|---|---|---|---|
| 6 2 5 | | | 1 |

| Gruppe 2: kompensiert | | | |
|---|---|---|---|
| 3 0 | | 0 | 1 |

| Anzahl der behandelten Augen | | | |
|---|---|---|---|
| GI. chron. simplex | postkontusionelles GI. | neovask. GI | Sekundärgl. bei i.o. Entz. |
| 18 | 2 | 7 | 2 |

| Gruppe 1: dekompensiert>21 mm Hg appl. | | | |
|---|---|---|---|
| 12 | 2 7 | | 1 |

| Gruppe 2: kompensiert | | | |
|---|---|---|---|
| 6 0 0 | | | 1 |

Folgende mittlere Druckwerte wurden vor der Behandlung gemessen:
(I) Drucklage der Gruppe der dekompensierten Glaukome bei Behandlung mit Timolol 0,5 % oder Kombinationstherapie:
   26,4 mmHg applanatorsich (Schwankungsbreite 3,6 mm Hg)
(II) Drucklage der kompensierten Glaukome bei Behandlung mit Timolol 0,5 %:
   19,2 mm Hg applanatorsich (Schwankungsbreite 1,6 mm Hg)

### Ergebnisse nach erfindungsgemäßer Behandlung:

In Gruppe 1 konnte in 11 Fällen der Druck auf unter 21 mm Hg applanatorisch durch die erfindungsgemäße Behandlung gesenkt werden. In Gruppe 2 gelang dies in allen 4 Fällen. Lediglich 2 Patienten mit einem G1. chron. simplex und 1 Patient mit einem neovaskulären Glaukom in Gruppe 1 konnten nicht reguliert werden. In Gruppe 3 konnte keine signifikante Drucksenkung festgestellt werden.

In 15 von 18 Fällen gelang also eine Druckregulierung mit der erfindungsgemäßen Kombination.

In all diesen 15 Fällen zeigte sich eine Überlegenheit gegenüber der zuvor durchgeführten Behandlung mit Timolol 0,5 % beziehungsweise einer zuvor durchgeführten Kombinationstherapie mit Timolol 0,5 % und einem weiteren drucksenkenden Mittel.

Von besonderem Interesse sind die 14 gegen Timolol 0,5 % resistent gewordenen Glaukome (Gruppe 1). Für diese Glaukome zeigt sich die Überlegenheit der erfindungsgemäßen Kombination gegenüber der 5-fachen Timololdosis bzw. Überlegenheit gegenüber einer Rombinationstherapie mit weiteren Antiglaukomatosa unter Einschluss von Timolol 0,5 %

## Patentansprüche

1. Pharmazeutisches Mittel, enthaltend wenigstens einen antiglaukomatösen Wirkstoff und Lactalbüminhydrolysat oder eine Lactalbuminhydrolysat-Peptid enthaltende Fraktion davon, wobei der antiglaukomatöse Wirkstoff ausgewählt ist unter Acetylcholinchlorid, Carbachol, Bethanicol, Pilocarpin, Aceclidinum, Eserin, Neostigmin, Galanthiamin, Brimonidin, Clonidin, Dipivefrin, Apraclonidin, Carteolol, Timolol, Metipranolol, Betaxolol, Befunolol, Pindolol, Levobunolol, Bupranolol, Brinzolamid, Dorzolamid, Acetazolamid, Methazolamid, Ethoxzolamid, Diclofenamid, Demecarium-Bromid, Diethyl-p-nitrophenylphosphat, Diisopropylfluorophosphat, Ecothiopat-Jodid und Derivaten davon, gegebenenfalls auch in Salzform.

2. Pharmazeutisches Mittel nach Anspruch 1, **dadurch gekennzeichnet, daß** das Lactalbuminhydrolysat oder die Fraktion davon ein gewichtsmittleres Molekulargewicht von etwa 50 bis 1200, vorzugsweise von etwa 60 bis 800 und insbesondere von etwa 80 bis 500 aufweist.

3. Pharmazeutisches Mittel nach einem der vorhergehenden Ansprüche, **dadurch gekennzeichnet, daß** das Lactalbuminhydrolysat erhältlich ist durch enzymatische Hydrolyse mit Papain, Pancreatin und wenigstens einer bakteriellen Protease.

4. Pharmazeutisches Mittel nach Anspruch 1, **dadurch gekennzeichnet, daß** die Fraktion des Lactalbuminhydrolysats erhältlich ist durch Extraktion eines Lactalbuminhydrolysats mit Ethanol und Gewinnung des Ethanolextrakts, gewünschtenfalls als Trokkenextrakt.

5. Pharmazeutisches Mittel nach Anspruch 1, **dadurch gekennzeichnet, daß** die Fraktion des Lactalbuminhydrolysats erhältlich ist durch Extraktion eines Lactalbuminhydrolysats mit Ethanol, Einengen des Extrakts zur Trockne, Extraktion des resultierenden Ethanol-Trockenextrakts mit Isopropanol und Gewinnung des Isopropanol-Extrakts, gewünschtenfalls als Trockenextrakt.

6. Pharmazeutisches Mittel nach Anspruch 1, **dadurch gekennzeichnet, daß** die Fraktion des Lactalbuminhydrolysats erhältlich ist durch Extraktion eines Lactalbuminhydrolysats mit Ethanol, Einengen des Extrakts zur Trockne, Extraktion des resultierenden Ethanol-Trockenextrakts mit Isopropanol, Einengen des Extrakts zur Trockne, Aufnahme des resultierenden Isopropanol-Trockenextrakts in Ethanol, Zugabe von Wasser bis auf ein Verhältnis von Ethanol:Wasser von etwa 20:80 bis 60:40, vorzugsweise von etwa 30:70 bis 50:50 und insbesondere von etwa 40:60, Abtrennen des Niederschlags und Gewinnen des wäßrig ethanolische Extrakts, gewünschtenfalls als Trockenextrakt.

7. Pharmazeutisches Mittel nach einem der vorhergehenden Ansprüche, **dadurch gekennzeichnet, daß** der antiglaukomatöse Wirkstoff ausgewählt ist unter Timolol sowie Derivaten davon, gegebenenfalls auch in Salzform.

8. Verwendung wenigstens eines antiglaukomatösen Wirkstoffs und von Lactalbuminhydrolysat oder einer Lactalbuminhydrolysat-Peptid enthaltenden Fraktion davon zur Herstellung eines pharmazeutischen Mittels zur Behandlung von Glaukomen, wobei der antiglaukomatöse Wirkstoff ausgewählt ist unter Acetylcholinchlorid, Carbachol, Bethanicol, Pilocarpin, Aceclidinum, Eserin, Neostigmin, Galanthiamin, Brimonidin, Clonidin, Dipivefrin, Apraclonidin, Carteolol, Timolol, Metipranolol, Betaxolol, Befunolol, Pindolol, Levobunolol, Bupranolol, Brinzolamid, Dorzolamid, Acetazolamid, Methazolamid, Ethoxzolamid, Diclofenamid, Demecarium-Bromid, Diethyl-p-nitrophenylphosphat, Diisopropylfluorophosphat, Ecothiopat-Jodid und Derivaten davon, gegebenenfalls auch in Salzform.

9. Verwendung nach Anspruch 8, wobei der antiglaukomatöse Wirkstoff und das Lactalbuminhydrolysat oder die Fraktion davon gleichzeitig oder zeitlich beabstandet, gemeinsam in einer Formulierung oder getrennt in wenigstens zwei verschiedenen Formulierungen verabreicht werden.

## Claims

1. A pharmaceutical composition comprising at least one antiglaucomatous active ingredient and lactalbumin hydrolysate or a fraction thereof which contains lactalbumin hydrolysate peptide, wherein the antiglaucomatous active ingredient is selected from acetylcholine chloride, carbachol, bethanicol, pilocarpine, aceclidinum, eserine, neostigmine, galantamine, brimonidine, clonidine, dipivefrine, apraclonidine, carteolol, timolol, metipranolol, betaxolol, befunolol, pindolol, levobunolol, bupranolol, brinzolamide, dorzolamide, acetazolamide, methazolamide, ethoxzolamide, diclofenamide, demecarium bromide, diethyl p-nitrophenyl phosphate, diisopropyl fluorophosphate, ecothiopate iodide and derivatives thereof, optionally also in salt form.

2. The pharmaceutical composition as claimed in claim 1, **characterized in that** the lactalbumin hydrolysate or the fraction thereof has a weight average molecular weight of approximately 50 to 1200, preferably of approximately 60 to 800 and in particular of approximately 80 to 500.

3. The pharmaceutical composition as claimed in any one of the preceding claims, **characterized in that** the lactalbumin hydrolysate is obtainable by enzymatic hydrolysis with papain, pancreatin and at least one bacterial protease.

4. The pharmaceutical composition as claimed in claim 1, **characterized in that** the lactalbumin hydrolysate fraction is obtainable by extraction of a lactalbumin hydrolysate with ethanol and obtaining the ethanol extract, if desired as dry extract.

5. The pharmaceutical composition as claimed in claim 1, **characterized in that** the lactalbumin hydrolysate fraction is obtainable by extraction of a lactalbumin hydrolysate with ethanol, concentration of the extract to dryness, extraction of the resulting ethanol dry extract with isopropanol and obtaining the isopropanol extract, if desired as dry extract.

6. The pharmaceutical composition as claimed in claim 1, **characterized in that** the lactalbumin hydrolysate fraction is obtainable by extraction of a lactalbumin hydrolysate with ethanol, concentration of the extract to dryness, extraction of the resulting ethanol dry extract with isopropanol, concentration of the extract to dryness, taking up the resulting isopropanol dry extract in ethanol, addition of water until the ethanol:water ratio is approximately 20:80 to 60:40, preferably approximately 30:70 to 50:50 and in particular approximately 40:60, removal of the precipitate and obtaining the hydroethanolic extract, if desired as dry extract.

7. The pharmaceutical composition as claimed in any of the preceding claims, **characterized in that** the antiglaucomatous active ingredient is selected from timolol and derivatives thereof, optionally also in salt form.

8. The use of at least one antiglaucomatous active ingredient and lactalbumin hydrolysate or a fraction thereof which contains lactalbumin hydrolysate peptide for the preparation of a pharmaceutical composition for the treatment of glaucomas, wherein the antiglaucomatous active ingredient is selected from acetylcholine chloride, carbachol, bethanicol, pilocarpine, aceclidinum, eserine, neostigmine, galantamine, brimonidine, clonidine, dipivefrine, apraclonidine, carteolol, timolol, metipranolol, betaxolol, befunolol, pindolol, levobunolol, bupranolol, brinzolamide, dorzolamide, acetazolamide, methazolamide, ethoxzolamide, diclofenamide, demecarium bromide, diethyl p-nitrophenyl phosphate, diisopropyl fluorophosphate, ecothiopate iodide and derivatives thereof, optionally also in salt form.

9. The use of claim 8, wherein the antiglaucomatous active ingredient and the lactalbumin hydrolysate or the fraction thereof are administered simultaneously or with a time lag, together in one formulation or separately in at least two different formulations.

## Revendications

1. Agent pharmaceutique, contenant au moins un agent actif antiglaucomateux et un hydrolysat de lactalbumine ou une de ses fractions contenant un peptide d'hydrolysat de lactalbumine, l'agent actif antiglaucomateux étant choisi parmi le chlorure d'acétylcholine, le carbachol, le béthanicol, la pilocarpine, l'acéclidine, l'ésérine, la néostigmine, la galanthiamine, la brimonidine, la clonidine, la dipivéfrine, l'apraclonidine, le cartéolol, le timolol, le métipranolol, le bétaxolol, le béfunolol, le pindolol, le lévobunolol, le bupranolol, le brinzolamide, le dorzolamide, l'acétazolamide, le méthazolamide, l'éthoxzolamide, le diclofénamide, le bromure de démêcarium, le phosphate de diéthyle et de p-nitrophényle, le fluorophosphate de diisopropyle, l'iodure d'écothiopate et leurs dérivés, le cas échéant également sous la forme de sel.

2. Agent pharmaceutique selon la revendication 1, **caractérisé en ce que** l'hydrolysat de lactalbumine ou sa fraction présente un poids moléculaire moyen en poids d'environ 50 à 1200, de préférence d'environ 60 à 800 et en particulier d'environ 80 à 500.

3. Agent pharmaceutique selon l'une quelconque des revendications précédentes, **caractérisé en ce que** l'hydrolysat de lactalbumine peut être obtenu par hydrolyse enzymatique avec de la papaïne, de la pancréatine et au moins une protéase bactérienne.

4. Agent pharmaceutique selon la revendication 1, **caractérisé en ce que** la fraction de l'hydrolysat de lactalbumine peut être obtenue par extraction d'un hydrolysat de lactalbumine avec de l'éthanol et obtention de l'extrait éthanolique, si on le souhaite en tant qu'extrait sec.

5. Agent pharmaceutique selon la revendication 1, **caractérisé en ce que** la fraction de l'hydrolysat de lactalbumine peut être obtenue par extraction d'un hydrolysat de lactalbumine avec de l'éthanol, par concentration de l'extrait jusqu'à dessiccation, extraction de l'extrait sec éthanolique qui en résulte avec de l'isopropanol et obtention de l'extrait isopropanolique, si on le souhaite en tant qu'extrait sec.

6. Agent pharmaceutique selon la revendication 1, **caractérisé en ce que** la fraction de l'hydrolysat de lactalbumine peut être obtenue par extraction d'un hydrolysat de lactalbumine avec de l'éthanol, par concentration de l'extrait jusqu'à dessiccation, extraction de l'extrait sec éthanolique qui en résulte avec de l'isopropanol, concentration de l'extrait à dessiccation, recueil de l'extrait sec isopropanolique qui en résulte dans de l'éthanol, addition d'eau jusqu'à un rapport éthanol/eau d'environ 20:80 à 60:40, de préférence d'environ 30:70 à 50:50 et, en particulier, d'environ 40:60, séparation du précipité et obtention de l'extrait éthanolique aqueux, si on le souhaite en tant qu'extrait sec.

7. Agent pharmaceutique selon l'une quelconque des revendications précédentes, **caractérisé en ce que** l'agent actif antiglaucomateux est choisi parmi le timolol ainsi que ses dérivés, le cas échéant aussi sous la forme de sel.

8. Utilisation d'au moins un agent actif antiglaucomateux et d'hydrolysat de lactalbumine ou d'une de ses fractions contenant un peptide d'hydrolysat de lactalbumine pour fabriquer un agent pharmaceutique pour traiter les glaucomes, l'agent actif antiglaucomateux étant choisi parmi le chlorure d'acétylcholine, le carbachol, le béthanicol, la pilocarpine, l'acéclidine, l'ésérine, la néostigmine, la galanthiamine, la brimonidine, la clonidine, la dipivéfrine, l'apraclonidine, le cartéolol, le timolol, le métipranolol, le bétaxolol, le béfunolol, le pindolol, le lévobunolol, le bupranolol, le brinzolamide, le dorzolamide, l'acétazolamide, le méthazolamide, l'éthoxzolamide, le diclofénamide, le bromure de démécarium, le phosphate de diéthyle et de p-nitrophényle, le fluorophosphate de diisopropyle, l'iodure d'écothiopate et leurs dérivés, le cas échéant également sous la forme de sel.

9. Utilisation selon la revendication 8, l'agent actif antiglaucomateux et l'hydrolysat de lactalbumine ou bien sa fraction sont administrés en même temps ou à distance l'un de l'autre, ensemble dans une formulation ou séparément dans au moins deux formulations différentes.
